(19) Europäisches Patentamt
European Patent Office
Office européen des brevets

(11) **EP 3 808 356 A1**

(12) **EUROPEAN PATENT APPLICATION**
published in accordance with Art. 153(4) EPC

(43) Date of publication:
**21.04.2021 Bulletin 2021/16**

(51) Int Cl.:
*A61K 35/28* (2015.01)  *A61K 35/35* (2015.01)
*A61K 35/50* (2015.01)  *A61K 35/51* (2015.01)
*A61L 27/36* (2006.01)  *A61L 27/38* (2006.01)
*A61L 27/40* (2006.01)  *A61P 17/02* (2006.01)

(21) Application number: **19818739.5**

(22) Date of filing: **17.06.2019**

(86) International application number:
**PCT/JP2019/023873**

(87) International publication number:
**WO 2019/240296 (19.12.2019 Gazette 2019/51)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **15.06.2018 JP 2018114800**

(71) Applicants:
• **Osaka Air Machine Service, Ltd.
Higashiosaka
Osaka 577-0058 (JP)**

• **Regene Pharm Co., Ltd.
Higashiosaka-shi, Osaka 577-0058 (JP)**

(72) Inventors:
• **MATSUYAMA, Akifumi
Higashiosaka-shi, Osaka 577-0058 (JP)**
• **OKURA, Hanayuki
Higashiosaka-shi, Osaka 577-0058 (JP)**

(74) Representative: **Moré, Solveig Helga et al
Kroher Strobel
Rechts- und Patentanwälte PartmbB
Bavariaring 20
80336 München (DE)**

(54) **TISSUE THERAPEUTIC AGENT**

(57) The present invention provides a pharmaceutical composition for healing tissue, said pharmaceutical composition comprising adherent cells originating from mesenchymal tissue treated with a physiologically active polypeptide or an LPS, or culture supernatant thereof, and a pharmaceutically acceptable carrier, and a method for producing the pharmaceutical composition.

[Fig. 5]

EP 3 808 356 A1

**Description**

Technical Field

The present invention relates to a pharmaceutical composition for tissue healing, and a method for producing the same. In particular, the present invention relates to a tissue healing agent containing drug-treated adherent cells derived from mesenchymal tissue or culture supernatant thereof, and a method for producing the same.

Background Art

Mesenchymal tissue-derived cells have been shown to be useful for tissue healing. Among them, mesenchymal stem cells (MSCs) are being actively studied for their clinical application in regenerative medicine. For example, tissue is considered as a source of stem cells (ASCs) (Non-Patent Document 1), and ASCs are known to have therapeutic effects in various areas (Non-Patent Document 2). In addition, adipose tissue-derived multilineage progenitor cells (ADMPCs) have also been shown to be effective for treatment of liver diseases (Patent Document 1).
Thus, mesenchymal tissue-derived cells have been shown to be useful in regenerative medicine involving tissue healing. Accordingly, further improvement of their healing ability is desired.

Prior Art Documents

Patent Document

Patent Document 1: WO 2008/153179

Non-Patent Documents

Non-Patent Document 1: Zuk PA, Zhu M, Ashjian P, et al. Human adipose tissue is a source of multipotent stem cells. Mol Biol Cell 2002; 13: 4279-4295.
Non-Patent Document 2: Japanese Journal of Transfusion and Cell Therapy, Vol. 59, No. 3: 450-456, 2013

Summary of the Invention

Problem to be solved by the Invention

It is an object to further improve the tissue healing ability of mesenchymal tissue-derived cells.

Solutions to the Problem

As a result of intensive studies to solve the above problems, the present inventors have found that adherent cells derived from mesenchymal tissue treated with a physiologically active polypeptide or LPS (lipopolysaccharide), or culture supernatant thereof have extremely high tissue healing ability, and completed the present invention.
That is, the present invention provides the followings:

(1) A pharmaceutical composition for tissue healing, including adherent cells derived from mesenchymal tissue treated with a physiologically active polypeptide or LPS, or culture supernatant thereof, and a pharmaceutically acceptable carrier.
(2) The pharmaceutical composition according to (1), wherein the physiologically active polypeptide is one or more polypeptides selected from the group consisting of inflammatory cytokine, inflammatory cytokine-inducing polypeptide, growth factor, chemokine, hormone and interferon.
(3) The pharmaceutical composition according to (1) or (2), wherein the physiologically active polypeptide is one or more polypeptides selected from the group consisting of interferon-β(IFN-β), interferon gamma (IFNγ), interleukin-1 alpha (IL-1α), interleukin-1 beta (IL-1β), interleukin-17A (IL-17A), tumor necrosis factor alpha (TNFa), tumor necrosis factor beta (TNFβ), type I interferon (INF-I), transforming growth factor β (TGFβ), epidermal growth factor (EGF) and fibroblast growth factor (FGF).
(4) The pharmaceutical composition according to any one of (1) to (3), wherein the adherent cells derived from mesenchymal tissue are mesenchymal tissue-derived stem cells (MSCs), adipose tissue-derived multilineage progenitor cells (ADMPCs), placenta tissue-derived cells, umbilical cord tissue-derived cells, cells derived from tissue of placenta and its appendages, or bone marrow tissue- or synovium tissue-derived cells.

(5) The pharmaceutical composition according to any one of (1) to (4), wherein the tissue healing is tissue protection, repair of tissue/cell injury, promotion of proliferation of cells constituting a tissue, suppression of tissue inflammation or reconstruction of tissue form.

(6) The pharmaceutical composition according to any one of (1) to (5), wherein the tissue healing is tissue healing in chronic phase disease.

(7) A method for producing a pharmaceutical composition for tissue healing, including the steps of:

(a) treating adherent cells derived from mesenchymal tissue with a physiologically active polypeptide or LPS, and
(b) mixing the cells treated in step (a) or culture supernatant thereof with a pharmaceutically acceptable carrier.

(8) The method according (7), wherein the physiologically active polypeptide is one or more polypeptides selected from the group consisting of inflammatory cytokine, inflammatory cytokine-inducing polypeptide, growth factor, chemokine, hormone and interferon.

(9) The method according to (7) or (8), wherein the physiologically active polypeptide is one or more polypeptides selected from the group consisting of interferon-$\beta$ (IFN-$\beta$), interferon gamma (IFN$\gamma$), interleukin-1 alpha (IL-1$\alpha$), interleukin-1 beta (IL-1$\beta$), interleukin-17A (IL-17A), tumor necrosis factor alpha (TNFa), tumor necrosis factor beta (TNP$\beta$), type I interferon (INF-I), transforming growth factor $\beta$ (TGF$\beta$), epidermal growth factor (EGF) and fibroblast growth factor (FGF).

(10) The method according to any one of (7) to (9), wherein the adherent cells derived from mesenchymal tissue are mesenchymal tissue-derived stem cells (MSCs), adipose tissue-derived multilineage progenitor cells (ADMPCs), placenta tissue-derived cells, umbilical cord tissue-derived cells, cells derived from tissue of placenta and its appendages, or bone marrow tissue- or synovium tissue-derived cells.

(11) The method according to any one of (7) to (10), wherein the tissue healing is tissue protection, repair of tissue/cell injury, promotion of proliferation of cells constituting a tissue, suppression of tissue inflammation, wound healing, or reconstruction of tissue form.

(12) The method according to any one of (7) to (11), wherein the tissue healing is tissue healing in chronic phase disease.

Effects of the Invention

According to the present invention, a pharmaceutical composition having an extremely high tissue healing ability can be obtained. The pharmaceutical composition of the present invention is useful for tissue healing in chronic phase-tissue injury and the like.

Brief Description of Drawings

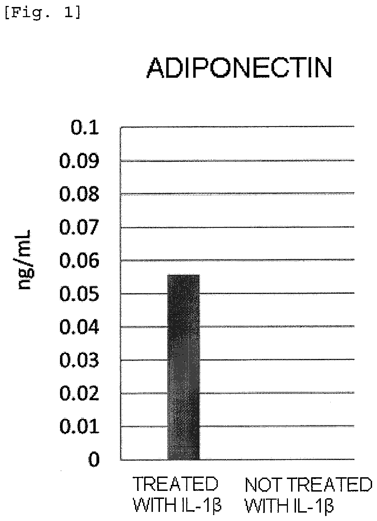

Fig. 1 is a graph comparing the produced amount of adiponectin in adipose tissue-derived multilineage progenitor cells (ADMPCs) treated with IL-1$\beta$ (left) and the produced amount of adiponectin in ADMPCs not treated with IL-1$\beta$ (right). The vertical axis represents the amount of adiponectin produced.

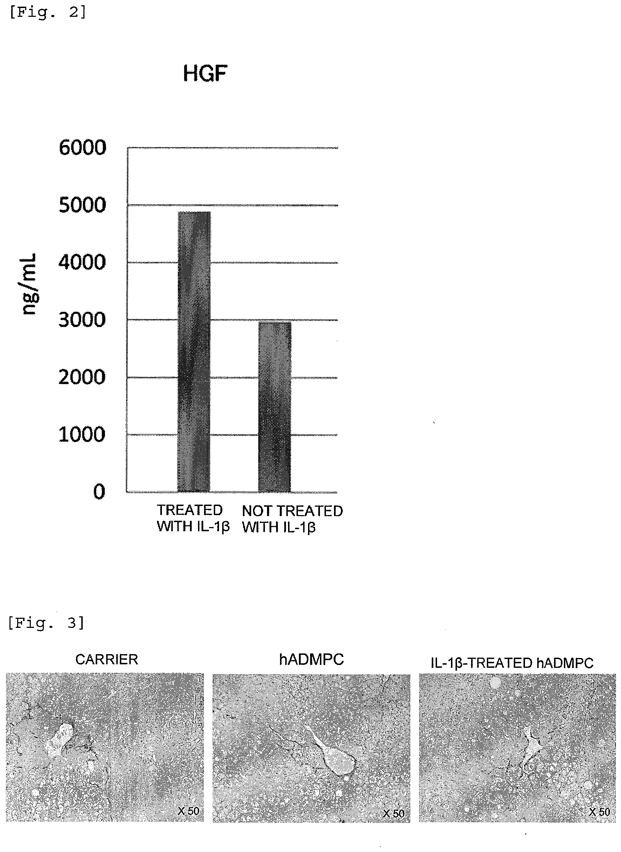

Fig. 2 is a graph comparing the produced amount of hepatocyte growth factor (HGF) in ADMPCs treated with IL-1$\beta$ (left) and the produced amount of HGF in ADMPCs not treated with IL-1$\beta$ (right). The vertical axis represents the amount of HGF produced.

Fig. 3 is an image of a Sirius red-stained tissue section showing decrease in intrahepatic fibers by ADMPCs treated with IL-1$\beta$ in non-alcoholic steatohepatitis (NASH) model mice. The result of a carrier administered group is in the left, the result of an administrated group with ADMPCs not treated with IL-1$\beta$ is in the middle, and the result of an administered group with ADMPCs treated with IL-1$\beta$ is in the right. The magnification is 50 times.

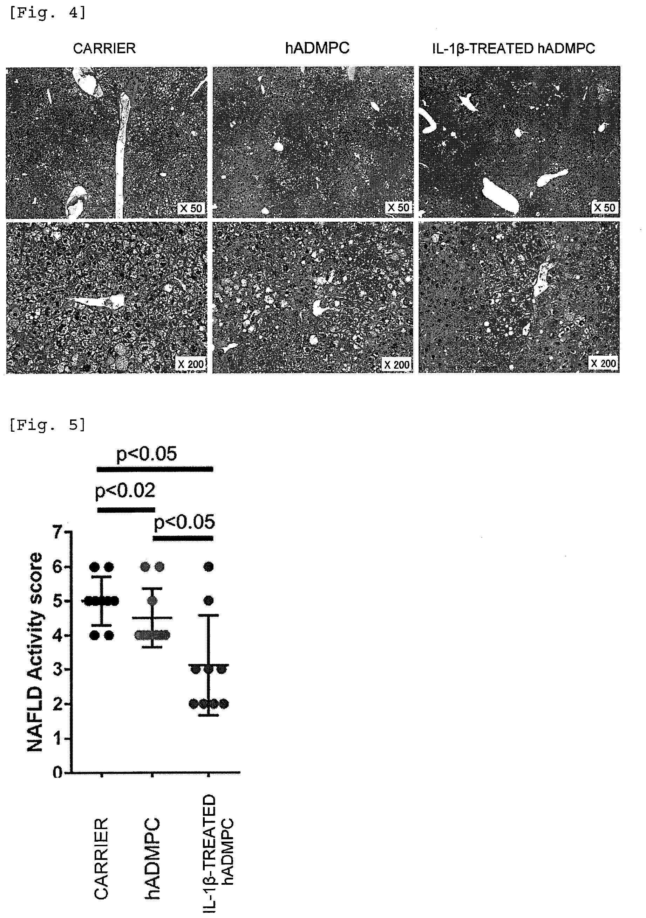

Fig. 4 is an image of a HE-stained tissue section showing reduction in liver tissue injury by ADMPCs treated with IL-1$\beta$ in non-alcoholic steatohepatitis (NASH) model mice. The result of a carrier administered group is in the left, the result of an administrated group with ADMPCs not treated with IL-1$\beta$ is in the middle, and the result of an administered group with ADMPCs treated with IL-1$\beta$ is in the right. The magnification on the top panels is 50 times, and the magnification on the bottom panels is 200 times.

Fig. 5 is a graph comparing reduction in liver tissue injury by ADMPCs treated with IL-1$\beta$ and ADMPCs not treated with IL-1$\beta$ in non-alcoholic steatohepatitis (NASH) model mice using NAFLD Activity Score. The p value is according to Mann-Whitney's U test.

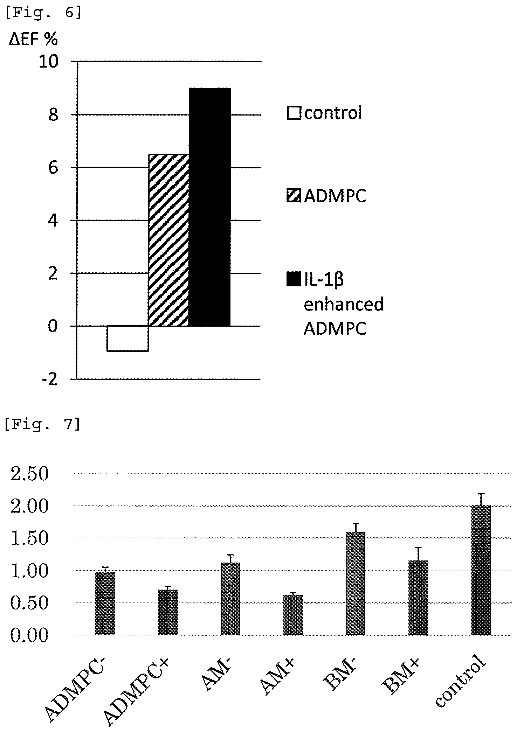

Fig. 6 is a graph showing improvement in left ventricular ejection fraction by ADMPCs treated with IL-1β and ADMPCs not treated with IL-1β in severe myocardial infarction model animals (pigs). The vertical axis (ΔEF%) represents change (%) in left ventricular ejection fraction before and after administration of cells. The white bar represents a control group to which cells are not administered, the hatched bar represents a group to which ADMPCs not treated with IL-1β are administered, and the black bar represents a group to which ADMPCs treated with IL-1β are administered.

Fig. 7 is a graph showing investigation results of reduction of liver tissue injury in chronic hepatitis model animals (rats) by ADMPCs treated with IL-1β (ADMPC+), placenta-derived cells treated with IL-1β (AM+), and bone marrow-derived mesenchymal stem cells treated with IL-1β (BM+), and ADMPCs not treated with IL-1β (ADMPC-), and bone marrow-derived mesenchymal stem cells not treated with IL-1β (BM-). The vertical axis shows areas of Sirius Red staining regions (fibrosis region). p-values are as follows: ADMPC- vs. ADMPC+ 0.018, BM- vs. BM+ 0.016, AM- vs. AM+ 0.032, control vs. ADMPC- 0.008, control vs. BM- no significance, control vs. AM-0.022, control vs. ADMPC+ 0.004, control vs. BM+ 0.056, control vs. AM+ 0.012.

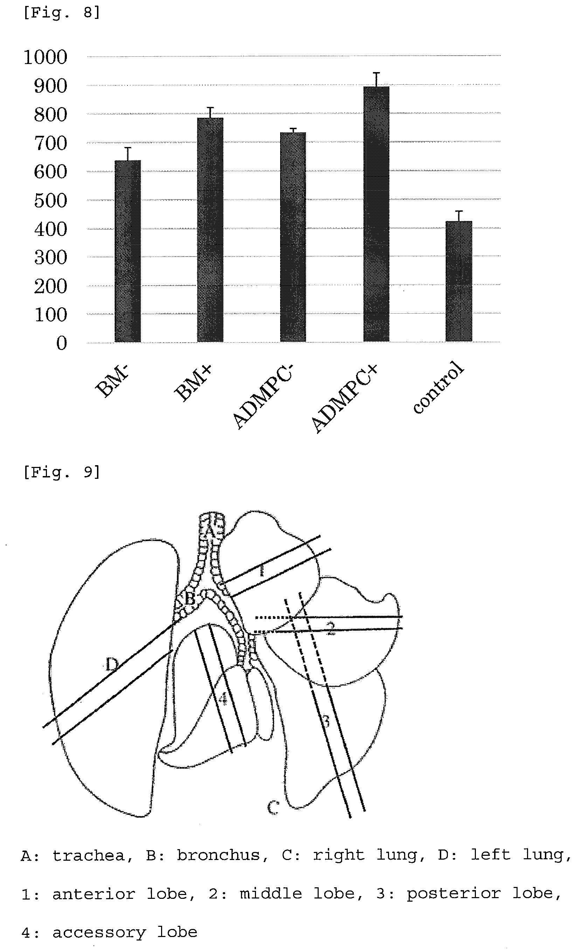

Fig. 8 is a graph showing investigation results of treatment effect of cardiac tissue in severe myocardial infraction model animals (nude rats) by bone marrow-derived mesenchymal stem cells treated with IL-1β (BM+), ADMPCs treated with IL-1β (ADMPC+), bone marrow-derived mesenchymal stem cells not treated with IL-1β (BM-), and ADMPCs not treated with IL-1β (ADMPC-). The vertical axis shows myocardial thickness after myocardial infraction. p-values are as follows: control vs. BM- 0.022479, control vs. BM+ 0.008113, BM- vs. BM+ 0.045328, cvontrol vs. ADMPC- 0.008113, control vs. ADMPC+ 0.019964, ADMPC- vs. ADMPC+ 0.014214.

Fig. 9 is a drawing showing cutting sections of lungs fixed with formalin in Example 8.

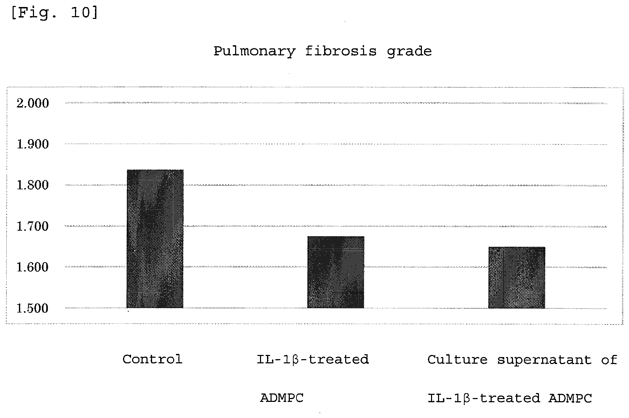

Fig. 10 is a graph showing investigation results of treatment effects of lung tissue in pulmonary fibrosis model animals (mice) by ADMPCs treated with IL-1β, culture supernatant thereof, and saline. The vertical axis shows pulmonary fibrosis scores.

Mode for Carrying Out the Invention

In one aspect, the present invention provides a pharmaceutical composition for tissue healing, including adherent cells derived from mesenchymal tissue treated with a physiologically active polypeptide or LPS, or culture supernatant thereof, and a pharmaceutically acceptable carrier. Here, the physiologically active polypeptide is a polypeptide that acts on a certain physiological regulatory function of the living body. Polypeptide refers to a substance in which two or more amino acid residues are linked to each other via a peptide bond. Various types of LPS are known, and any LPS may be used.

The physiologically active polypeptide used in the present invention also includes its variants. The variant of the physiologically active polypeptide is one having an activity capable of, when acted on mesenchymal tissue-derived adherent cells, providing mesenchymal tissue-derived adherent cells or culture supernatant thereof that can be used for tissue healing of the present invention.

The variant refers to a polypeptide in which the amino acid residue constituting the polypeptide has been substituted, deleted or added, with respect to the original peptide. The number of amino acid residues to be substituted, deleted or added is not particularly limited. For example, one to several amino acid residues may be substituted, deleted or added. For example, the variant polypeptide may have an amino acid sequence identity of 80% or more, preferably 90% or more, for example 95% or more, 97% or more, or 99% or more, with respect to the original polypeptide. Furthermore, the variant of the physiologically active polypeptide may be one in which the amino acid residue constituting the polypeptide is modified. The modification may be with any type of label. The modification may be chemical modification such as methylation, halogenation or glycosylation, or labeling such as fluorescence labeling or radioactive labeling. The variant of the physiologically active polypeptide may be one in which some amino acid residues are linked to each other via a bond other than a peptide bond.

The physiologically active polypeptide used in the present invention may be any polypeptide. Suitable physiologically active polypeptides used in the present invention are preferably cytokine, in particular one or more polypeptides selected from the group consisting of inflammatory cytokine, inflammatory cytokine-inducing polypeptide, growth factor, hormone and interferon. The inflammatory cytokine is a cytokine involved in pathogenesis of inflammation. The inflammatory cytokine-inducing polypeptide is a polypeptide having an effect of increasing the amount of inflammatory cytokine or enhancing the activity thereof. The growth factor is a polypeptide that promotes the growth or differentiation of specific cells in vivo. The chemokine is a fundamental protein that exhibits the action via a G protein coupled receptor and is a group of cytokines. The hormone is a substance that is produced in vivo, transported via body fluids, and affects the activity of specific cells, tissue or organ. The interferon is a group of cytokines produced in response to entry of foreign substances such as virus, pathogen or tumor cells in vivo. Various inflammatory cytokines, inflammatory cytokine-inducing polypeptides, growth factors and interferons are publicly known and any of them may be used.

The cytokines include, but are not limited to, IL-1α, IL-1β, IL-2 to IL-35, OSM (Oncostatin M), LIF, CNTF, CT-

1, TNF-$\alpha$, TNF-$\beta$, BAFF, FasL, RANKL and TRAIL. The inflammatory cytokines include, but are not limited to, IL-1$\alpha$, IL-1$\beta$, IL-6, IL-8, IL-12, IL-18 and TNF$\alpha$.

The inflammatory cytokine-inducing polypeptides include, but are not limited to, IL-17A.

The growth factors include, but are not limited to, activin A, ANGPTL5, BAFF, BD-2, BD-3, BNDF, BMP-1 to 7, DKK1, EGF, EG-VEGF, FGF-1 to 21, G-CSF, GM-CSF, HGF, IGF-1, IGF-2, platelet-derived growth factor (PDGF)-AA, PDGF-AB, PDGF-BB, R-spondin-1 to 3, SCF, galectin-1 to 3, GDF-11, GDNF, pleiotrophin, TGF-$\alpha$, TGF-$\beta$, TPO (thrombopoietin), TSLP, vascular endothelial growth factor (VEGF) and ciliary neurotrophic factor (CNTF).

The chemokines include, but are not limited to, CCL1 to CCL28 and CXCL1 to CXCL10.

The hormones include, but are not limited to, Calcitonin, Parathormone, Glucagon, Erythropoietin, Leptin, ANP, BNP, CNP, Oxytocin, Vasopressin, TRH (thyrotropin releasing hormone), TSH (thyroid stimulating hormone), CRH (corticotropin releasing hormone), ACTH (adrenocorticotropin hormone), GRH (gonadotropin releasing hormone), FSH (follicle stimulating hormone), LH (luteinizing hormone), SOM (somatostatin), GRH (growth hormone releasing hormone), GH (growth hormone), PRH (prolactin releasing hormone), PIH (prolactin inhibiting hormone) and Prolactin.

The interferons include, but are not limited to, IFN-$\alpha$, IFN-$\beta$, IFN-$\gamma$ and IFN-I.

Suitable physiologically active peptides used in the present invention are inflammatory cytokine, inflammatory cytokine-inducing polypeptide, growth factor and interferon. Among them, preferable examples include, but are not limited to, IFN-$\beta$, IFN-$\gamma$, IL-1$\alpha$, IL-1$\beta$, IL-17A, TNF$\alpha$, TNF-$\beta$, INF-I, TGF$\beta$, EGF and FGF.

The tissue healing refers to restoring a tissue to a normal state or bringing a tissue closer into a normal state, including tissue protection, repair of tissue/cell injury, promotion of proliferation of cells constituting a tissue, suppression of tissue inflammation, wound healing, and reconstruction of the tissue form. Because the cells or culture supernatant thereof in the pharmaceutical composition of the present invention are useful for tissue protection, promotion of proliferation of cells constituting a tissue, etc., the pharmaceutical composition of the present invention is preferably used for tissue healing in chronic phase disease.

Tissues to be healed by the pharmaceutical composition of the present invention are any tissue of animal and are not particularly limited. Examples of the tissues include, but are not limited to, liver, pancreas, kidney, muscle, bone, cartilage, bone marrow, stomach, intestine, blood, nerve, skin, mucous membrane, heart and hair. Suitable tissues to be healed by the pharmaceutical composition of the present invention are liver, nerve, skin, mucous membrane and heart. Therefore, the pharmaceutical composition of the present invention is preferably used for treatment of, for example, liver cirrhosis, hepatitis and NASH (nonalcoholic steatohepatitis), and is also effective for chronic phase disease.

The cells or culture supernatant thereof that are an active ingredient of the pharmaceutical composition of the present invention are adherent cells derived from mesenchymal tissue treated with a physiologically active polypeptide or LPS, or culture supernatant thereof.

The pharmaceutical composition of the present invention may be administered to a subject of the same animal species as, or different animal species from which the active ingredient cells or culture supernatant thereof are derived. For example, the pharmaceutical composition of the present invention including adherent cells derived from human-derived mesenchymal tissue treated with an inflammatory cytokine-inducing agent or culture supernatant thereof may be administered to a human subject. The cells or culture supernatant thereof in the pharmaceutical composition of the present invention may be from the same human subject as the human subject to be administered, or may be from a different human subject from the human subject to be administered.

Any mesenchymal tissue-derived adherent cells may be used in the present invention. The mesenchymal tissue-derived adherent cells may be commercially available ones, for example, distributed ones from organizations such as American Type Culture Collection (ATCC) (US) and NITE (Japan). Alternatively, mesenchymal tissue-derived adherent cells may be obtained from mesenchymal tissue. Means and methods for preparing mesenchymal tissue-derived adherent cells from mesenchymal tissue are publicly known.

Examples of suitable mesenchymal tissue-derived adherent cells include, but are not limited to, mesenchymal tissue-derived stem cells (MSCs) such as adipose tissue-derived stem cells (ASCs), adipose tissue-derived multilineage progenitor cells (ADMPCs), Muse cells, cells derived from bone marrow tissue, placenta tissue, umbilical cord tissue, amniotic tissue, cartilage tissue, periosteum tissue, synovium tissue, skeletal muscle tissue, and tissue of placenta and its appendages, stem cells, stromal cells, and menstrual blood cells.

When the cells are obtained from mesenchymal tissue, they may be isolated from any mesenchymal tissue. Examples of mesenchymal tissue include, but are not limited to, adipose tissue, bone marrow tissue, placenta tissue, umbilical cord tissue, amniotic tissue, cartilage tissue, periosteum tissue, synovium tissue, skeletal muscle tissue, placenta tissue and menstrual blood. Preferable mesenchymal tissues include adipose tissue, bone marrow tissue, synovium tissue, placenta tissue, umbilical cord tissue, and tissue of placenta and its appendages. In particular, adipose tissue is preferable because it is contained in a large amount in the body and many cells can be extracted.

Adherent cells can be obtained by extracting mesenchymal tissue from the body, placing and culturing the tissue in a culture vessel, and selectively acquiring cells adhering to the vessel. Mesenchymal tissue can be extracted using publicly known means and methods such as excision and aspiration. The extracted mesenchymal tissue may be cultured as it is, or if necessary, the extracted mesenchymal tissue may be minced or broken, followed by removing of red blood cells to culture the obtained cell population. These treating methods and means, and cell culturing means and methods are publicly known, and can be appropriately selected. Mesenchymal tissue-derived adherent cells may be obtained, for example, by treating the cells attached to the culture vessel with an enzyme such as trypsin.

Treatment of mesenchymal tissue-derived adherent cells with a physiologically active polypeptide or LPS may be carried out by contacting the cells with cytokine in a publicly known manner. Typically, this treatment may be carried out by culturing mesenchymal tissue-derived adherent cells for a certain period of time in a medium containing an appropriate concentration of physiologically active polypeptide or LPS. Usually, mesenchymal tissue-derived adherent cells are cultured in several nanograms/ml to several hundred nanograms/ml of inflammatory cytokine or a medium to which inflammatory cytokine has been added. The medium for use in culture may be a publicly known one. The culturing time and culturing temperature may also be appropriately selected. If necessary, mesenchymal tissue-derived adherent cells may be cultured to increase the number of cells, before treatment with a physiologically active polypeptide or LPS. A desired subpopulation may be obtained from a population of mesenchymal tissue-derived adherent cells, and if necessary, the subpopulation may be cultured to increase the number of cells, before treatment with a physiologically active polypeptide or LPS.

The number of types of physiologically active polypeptide or LPS for use in treatment of mesenchymal tissue-derived adherent cells may be one or two or more.

Mesenchymal tissue-derived adherent cells treated with a physiologically active polypeptide or LPS increase expression and production of one or more factors that contribute to tissue repair (such as polypeptide, growth factor and/or enzyme involved in tissue healing), or become to express and produce the same. Such factors include, but are not limited to, adiponectin, HGF, CSF2 (GM-CSF), CSF3 (G-CSF), LIF, MMP family factors, FGF family factors, ADAM family factors, angiopoietin-like protein family factors, pleiotrophin, R-spondin family factors and VEGF family factors. CSF2 or CSF3 contributes not only to activation of hematopoietic stem cells but also to stem cell proliferation and/or angiogenesis in many tissues or organs including brain, heart, lung, and liver, thereby contributing to tissue repair. Accordingly, cells that express and produce these factors at higher level are preferred. The increase in expression or production of the above factor may be, for example, 10 times or more, preferably 30 times or more, more preferably 50 times or more, still more preferably 100 times or more, compared to that before treatment.

Culture supernatant of mesenchymal tissue-derived adhesive cells treated with a physiologically active polypeptide or LPS may be obtained by methods described below.

(1) culturing mesenchymal tissue-derived adherent cells in a medium containing a physiologically active polypeptide or LPS, and obtaining culture supernatant thereof.
(2) culturing mesenchymal tissue-derived adherent cells in a medium containing a physiologically active polypeptide or LPS, transferring the cultured cells to another medium, culturing them, and obtaining culture supernatant thereof.

Culture in a medium containing a physiologically active polypeptide or LPS may be performed in a similar manner to the treatment with a physiologically active polypeptide or LPS as described above. Conditions of cell culture such as medium components, culture time, culture temperature can be appropriately selected and changed according to cell type, cell number needed, use of cells, and so on of cells. Culture supernatant may be concentrated using a known method for concentration such as centrifugation, filtration by a filter, precipitation with ammonium sulfate, freeze-drying, and so on.

The pharmaceutical composition of the present invention can be produced by mixing mesenchymal tissue-derived adherent cells treated with a physiologically active polypeptide or LPS, or culture supernatant thereof as described above with a pharmaceutically acceptable carrier. A variety of pharmaceutically acceptable carriers are publicly known and may be appropriately selected for use. For example, when the pharmaceutical composition of the present invention is used as an injection, the cells may be suspended in a carrier such as purified water, saline or phosphate buffered saline. The pharmaceutical composition of the present invention comprising culture supernatant is preferable in that there is no matter such as occlusion of blood vessels and needles with cells when administered.

The dosage form of the pharmaceutical composition of the present invention is not particularly limited, but may be a solution, semisolid or solid. The administration method of the pharmaceutical composition of the present invention is also not limited, but may include local injection, intravenous injection or infusion, application to an affected area, administration to an affected area via a catheter, or direct transplantation to tissues such as liver by a surgical procedure. The pharmaceutical composition of the present invention may be transplanted in the form of cell sheet, cell mass, layered cell sheet, etc.

The administration route and dose of the pharmaceutical composition of the present invention may be appropriately determined in consideration of the type and site of the tissue to be healed, the degree of disease, the condition of the subject and the like.

The pharmaceutical composition of the present invention may contain cells other than adherent cells derived from mesenchymal tissue treated with a physiologically active polypeptide or LPS. The pharmaceutical composition of the present invention may contain components other than the culture supernatant of mesenchymal tissue-derived adhesive cells treated with a physiologically active polypeptide or LPS.

In a further aspect, the present invention provides use of adherent cells derived from mesenchymal tissue treated with a physiologically active polypeptide or LPS, or culture supernatant thereof in producing a medicament for tissue healing.

In a further aspect, the present invention provides use of adherent cells derived from mesenchymal tissue treated with a physiologically active polypeptide or LPS, or culture supernatant thereof for tissue healing.

In a further aspect, the present invention provides a method for tissue healing in a subject in need of tissue healing, including administering to the subject adherent cells derived from mesenchymal tissue treated with a physiologically active polypeptide or LPS, or culture supernatant thereof.

In yet another aspect, the present invention provides a method for producing a pharmaceutical composition for tissue healing, including the steps of:

(a) treating adherent cells derived from mesenchymal tissue with a physiologically active polypeptide or LPS, and
(b) mixing the cells treated in step (a) or culture supernatant thereof with a pharmaceutically acceptable carrier.

In yet another aspect, the present invention provides a method for producing cells for tissue healing, including treating adherent cells derived from mesenchymal tissue with a physiologically active polypeptide or LPS. In yet another aspect, the present invention provides a method for producing cell culture supernatant for tissue healing, which comprises treating mesenchymal tissue-derived adhesive cells with a physiologically active polypeptide or LPS, and obtaining culture supernatant thereof.

Hereinafter, more detailed and specific description is made of the present invention with reference to Examples, but the Examples are not intended to limit the present invention.

Example 1

Example 1. Effect of treating ADMPCs with IL-Iβ

(1) Method of experiment

(i) Collection of adipose tissue from human subject

From six women from which informed consent was obtained, extra adipose tissue to be discarded was received during liposuction surgery. The protocol conformed to the Kobe University Graduate School of Medicine Review Boards for Human Research.

(ii) Isolation and culture of ADMPCs

The adipose tissue was minced and then digested in Hanks' buffered saline solution (HBSS) containing 0.008% Liberase (Roche Lifescience) with shaking in a water bath at 37°C for 1 hour. The digested product was filtered through Cell Strainer (BD Bioscience), followed by centrifuging at 800 × g for 10 minutes. The lymphocyte separation solution (d = 1.077) (Nacalai tesque) was used to remove red blood cells by specific gravity method. The obtained cell population containing ADMPCs were seeded in DMEM containing 10% fetal bovine serum (Hyclone) to allow for attachment of the cells, followed by washing and treatment with EDTA to yield ADMPCs. Then, the ADMPCs in a medium (60% DMEM-low glucose, 40% MCDB201, 10 μg/mL EGF, 1 nM dexamethasone, 100 μM ascorbic acid and 5% FBS) were seeded on a human fibronectin-coated dish and subcultured 3 to 8 times to yield cultured ADMPCs.

(iii) IL-1β treatment

IL-Iβ was added to a medium (60% DMEM-low glucose, 40% MCDB 201, 10 μg/mL EGF, 1 nM dexamethasone, 100 μM ascorbic acid and 5% FBS) to a concentration of 10 ng/ml. The cultured ADMPCs obtained in (ii) above were cultured in the IL-1β-containing medium for 72 hours to measure adiponectin and hepatocyte growth factor (HGF) produced in the medium. The measurement of adiponectin was performed using the ELISA kit of abcam (Catalog No. ab99968). The measurement of HGF was performed using the ELISA kit of R & D System (Catalog No. DHG00). For a control, ADMPCs were cultured in the above medium except that IL-Iβ was not added.

(2) Results of experiment

The measurement results of amount of adiponectin produced are shown in Fig. 1. Production of adiponectin from ADMPCs not treated with IL-Iβ was not found, whereas it was confirmed that adiponectin was produced from ADMPCs treated with IL-1β.

The measurement results of amount of HGF produced are shown in Fig. 2. The amount of HGF produced from ADMPCs treated with IL-Iβ was increased by about 1.7 times as compared to the amount of HGF produced from ADMPCs not treated with IL-1β.

Example 2

Example 2. In vivo tissue healing effect of ADMPCs treated with IL-1β - alleviation of liver tissue injury

(1) Method of experiment

Collection of adipose tissue from a human subject, isolation and culture of ADMPCs, and IL-Iβ treatment were performed in the same manner as in Example 1, except that the concentration of IL-Iβ in the medium was 5 ng/ml.

ADMPCs treated with IL-1β were suspended in a carrier to a concentration of $1.2 \times 10^5$ cells/ml. These cells were administered to NASH model mice (STAM (registered trademark) mice) to examine healing of the liver tissue. The animals were divided into three groups: a group to which ADMPCs treated with IL-Iβ were administered (n = 9), a group to which ADMPCs not treated with IL-Iβ were administered (n = 9), and a group to which a carrier was administered (n = 10). At the beginning of the study, streptozotocin was administered to the animals in each group, and the animals were fed with a normal diet, fed with a high-fat diet from week 4 to week 9, and euthanized at week 9. Administration of ADMPCs ($3 \times 10^5$ cells/kg) and carrier was performed once at week 6. The liver tissue sections obtained were subjected to Sirius red staining and hematoxylin-eosin (HE) staining.

(2) Results of experiment

(i) Sirius red staining of liver tissue sections

The results of Sirius red staining of liver tissue sections from mice obtained at week 9 of the study are shown in Fig. 3. In the livers from mice to which ADMPCs treated with IL-1β was administered, it was confirmed that deposition of intrahepatic fibers stained with Sirius red was reduced, compared to the livers from carrier administered mice and mice to which ADMPCs not treated with IL-1β was administered.

(ii) HE staining of liver tissue sections

The results of HE staining of liver tissue sections from mice obtained at week 9 of the study are shown in Fig. 4. In the livers from mice to which ADMPCs treated with IL-1β was administered, it was confirmed that injury to liver tissue represented by vacuolation was reduced, compared to the livers from carrier administered mice and mice to which ADMPCs not treated with IL-1β was administered.

(iii) Evaluation of liver tissue healing effect by NAFLED activity score (E. M. Brunt et al. Hepatology. 2011 March; 53(3): 810-820)

The degree of liver tissue injury in mice obtained at week 9 of the study was evaluated according to the NAFLED activity score. The evaluation method of NAFLED activity score is shown in Table 1.

[Table 1]

EVALUATION METHOD OF NAFLD ACTIVITY SCORE

| ITEM | DEFINITION | SCORE |
| --- | --- | --- |
| FATTY CHANGE | FATTY CHANGE AT LOW TO MODERATE MAGNIFICATION | |
| | < 5 % | 0 |
| | 5-33% | 1 |
| | 33-66% | 2 |
| | > 66% | 3 |

(continued)

EVALUATION METHOD OF NAFLD ACTIVITY SCORE

| ITEM | DEFINITION | SCORE |
|---|---|---|
| INFLAMMATION OF LIVER PARENCHYMA | EVALUATION OF INFLAMMATION FOCI | |
| | NONE | 0 |
| | LESS THAN 2 SITES AT 200 TIMES ENLARGEMENT | 1 |
| | 2 TO 4 SITES AT 200 TIMES ENLARGEMENT | 2 |
| | MORE THAN 5 SITES AT 200 TIMES ENLARGEMENT | 3 |
| LIVER CELL INJURY (BALLOONING) | NONE | 0 |
| | 2 TO 3 BALLOONING CELLS | 1 |
| | 4 OR MORE BALLOONING CELLS | 2 |

The results are shown in Fig. 5. The NAFLD activity score was significantly lower in the livers from mice to which ADMPCs treated with IL-Iβ was administered, compared to the livers from carrier administered mice and mice to which ADMPCs not treated with IL-Iβ was administered. It was confirmed that injury to liver tissue represented by vacuolation, inflammation and fatty change was greatly reduced.

From these results, ADMPCs treated with IL-Iβ are found to be effective in healing injured tissue, and be useful for tissue protection, repair of tissue/cell injury, suppression of tissue inflammation and promotion of proliferation of cells constituting a tissue. It is considered that these tissue healings enables reconstruction of the tissue form and wound healing. Moreover, because these effects were observed in mice in which liver injury was induced by streptozotocin and high-fat diet, it can be said that IL-1β-treated ADMPCs are effective for tissue healing in chronic phase disease.

Example 3

Example 3. In vivo tissue healing effect of ADMPCs treated with IL-Iβ - improvement of cardiac function

(1) Method of experiment

A severe myocardial infarction model was created using 8 weeks old pigs by two-stage balooning/reperfusion method. In detail, a 6F guide catheter was transcutaneously placed through the femoral artery on the opening of the left coronary artery, a guide wire was inserted through the catheter into the first diagonal artery (# 9 in the AHA classification), and preconditioning was performed by conducting ballooning (obstruction reopening) with the aid of the guide. One week later, a guidewire was inserted into the left anterior descending coronary artery (# 6 to # 8 in the AHA classification), and ballooning (obstruction reopening) was performed at the left anterior descending coronary artery immediately below the bifurcation of the left circumflex coronary artery (# 6 in the AHA classification) to produce a myocardial ischemic region. Four weeks after that (five weeks after the first obstruction reopening), individuals with a cardiac ejection fraction of 40% or less in cardiac ultrasonography were subjected to the study as a severe heart failure model.

Four weeks after the second balooning/reperfusion, the animals were divided into following groups: a control group (cells were not administrated), a group to which non-activated cells (ADMPCs) were administered, and a group to which IL-Iβ activated cells (72 hours cultured) (IL-1β-activated ADMPCs) were administered. To cell administered groups, cells at a concentration of $3 \times 10^5$ cells/kg body weight were administered through a catheter via the coronary artery in the same manner. ADMPCs and IL-1β-activated ADMPCs were prepared in the same manner as in Example 1. Immediately before administration, cardiac MRI was performed 3 months after administration (Signa EXCITE XI TwinSpeed 1.5T Ver. 11.1, GE Healthcare), using Cardiac Vx (GE Healthcare) as analysis software, to measure left ventricular end-diastolic and end-systolic volumes.

The following formula:

```
Left ventricular ejection fraction = 100 × (left ventricular

end-diastolic volume - left ventricular end-systolic volume)

/ (left ventricular end-diastolic volume)
```

was used to calculate left ventricular ejection fraction (% EF). The difference between the value 3 months after administration and the value immediately before administration is represented as ΔEF (%) (Fig. 6).

(2) Results of experiment

As shown in Fig. 6, the left ventricular ejection fraction was decreased in the control group, whereas the left ventricular ejection fraction was improved in the two groups to which cells were administered, in particular, when IL-1β-activated cells were administered, the left ventricular ejection fraction was markedly improved.

These results indicate that IL-1β-treated ADMPCs heal cardiac tissue injured by severe myocardial infarction and markedly improve the cardiac function.

Example 4

Example 4. In vivo tissue healing effects of ADMPCs treated with IL-1β, bone marrow-derived mesenchymal stem cells treated with IL-1β, and placenta-derived cells treated with IL-Iβ - reduction of liver tissue fibrosis

(1) Method of experiment

(i) Collection of adipose tissue from a human subject

Extra adipose tissue to be discarded was provided during liposuction surgery from six women from whom informed consent was obtained. The protocol conformed to the Kobe University Graduate School of Medicine Review Boards for Human Research. Bone marrow-derived mesenchymal stem cells and placenta-derived cells were purchased from Lonza.

(ii) Isolation and culture of ADMPCs

The adipose tissue was minced and then digested in Hanks' buffered saline solution (HBSS) containing 0.008% Liberase (Roche Lifescience) in a water bath at 37°C for 1 hour with shaking. The digested product was filtered through Cell Strainer (BD Bioscience), followed by centrifuging at $800 \times g$ for 10 minutes. The lymphocyte separation solution (d = 1.077) (Nacalai tesque) was used to remove red blood cells by specific gravity method. The obtained cell population containing ADMPCs were seeded in DMEM containing 10% fetal bovine serum (Hyclone) to allow for attachment of the cells, followed by washing and treatment with EDTA to yield ADMPCs. Then, the ADMPCs in a medium (60% DMEM-low glucose, 40% MCDB201, 10 μg/mL EGF, 1 nM dexamethasone, 100 μM ascorbic acid and 5% FBS) were seeded on a human fibronectin-coated dish and subcultured 3 to 8 times to yield cultured ADMPCs.

Bone marrow-derived mesenchymal stem cells and placenta-derived cells were seeded to human fibronectin coated dishes with a medium (60% DMEM-low glucose, 40% MCDB201, 10 μg/mL EGF, 1nM dexamethasone, 100 μM ascorbic acid, and 5% FBS), and subcultured three times to subjected to experiments.

(iii) IL-1β treatment

IL-Iβ was added to a medium (60% DMEM-low glucose, 40% MCDB 201, 10 μg/mL EGF, 1 nM dexamethasone, 100 μM ascorbic acid and 5% FBS) to a concentration of 5 ng/ml. The cultured ADMPCs, bone marrow-derived mesenchymal stem cells, and placenta-derived cells obtained in section 2 above were cultured in the IL-1β-containing medium as described above for 72 hours. In the control system, ADMPCs, bone marrow-derived mesenchymal stem cells, and placenta-derived cells were cultured in the above medium without addition of IL-1β.

ADMPCs treated with IL-1β, bone marrow-derived mesenchymal stem cells treated with IL-1β, and placenta-derived cells treated with IL-Iβ were suspended in a carrier to a concentration of $1.2 \times 10^5$ cells/ml. These cells were administered to chronic hepatitis model animals made by a hepatitis-inducing agent administration to examine healing of the liver tissue. The animals were divided into 7 groups: a group to which ADMPCs treated with IL-Iβ were administered (n = 6), a group to which ADMPCs not treated with IL-Iβ were administered (n = 5), a group to which bone marrow-derived mesenchymal stem cells treated with IL-Iβ were administered (n = 5), a group to which bone marrow-derived mesenchymal stem cells not treated with IL-Iβ were administered (n = 4), a group to which placenta-derived cells treated with IL-Iβ were administered (n = 5), a group to which placenta-derived cells not treated with IL-1β were administered (n = 5), and a group to which a carrier was administered (n = 5).

Chronic hepatitis model animals were made by administration of a hepatitis-inducing agent. Chronic hepatitis model rats were obtained by intraperitoneal injection of 300μL/kg (each time) carbon tetrachloride twice a week for four weeks. ADMPCs, bone marrow-derived mesenchymal stem cells or placenta-derived cells ($3 \times 10^5$/kg) with/without IL-Iβ treatment, and a carrier were administered to tail vein of these rats. After one week from cell administration (after five week from the beginning of test), animals were euthanized under deep anesthesia, livers were removed, and fixed them with neutral buffered formalin. These liver samples were paraffin embedded, obtained thin slices, and the obtained liver tissue slices were subjected to Sirius Red staining.

(2) Results of experiments

(i) Sirius Red staining of liver tissue slices

Results of Sirius Red staining of rat liver tissue obtained on 5th week from the beginning of the test are shown in Fig. 7. Reduction of fiber deposition in the livers stained by Sirius Red was confirmed in the livers of rats to which ADMPCs treated with IL-1β, bone marrow-derived mesenchymal stem cells treated with IL-1β, or placenta-derived cells treated with IL-Iβ were administered, compared with the livers of rats to which ADMPCs not treated with IL-1β, bone marrow-derived mesenchymal stem cells not treated with IL-1β, or placenta-derived cells not treated with IL-1β were administered.

From these results, it was found that ADMPCs treated with IL-1β, bone marrow-derived mesenchymal stem cells treated with IL-1β, or placenta-derived cells treated with IL-Iβ are effective in healing injured tissue. It is considered that these tissue healing enables reconstruction of the tissue form and wound healing. Moreover, because these effects were found in chronic hepatitis model animals made by administration of a hepatitis-inducing agent administration, it can be said that ADMPCs treated with IL-1β, bone marrow-derived mesenchymal stem cells treated with IL-1β, or placenta-derived cells treated with IL-Iβ are effective in tissue healing in chronic phase disease.

Example 5

Example 5. In vivo tissue healing effect of ADMPCs treated with IL-1β or bone-marrow-derived mesenchymal stem cells treated with IL-Iβ - improvement of cardiac tissue

(1) Method of experiment

Chest of an 8-week-old nude rat deeply anesthetized with a general anesthetic was opened to expose the heart, and the descending branch of the coronary artery was completely ligated to create an acute myocardial infraction model. Two weeks later, two IL-1β-treated ADMPC sheets or two IL-1β-treated bone-marrow-derived mesenchymal stem cell sheets were transplanted to the left ventricular wall of the myocardial infraction model rat under general anesthesia with left intercostal thoracotomy. Four weeks after transplantation, the animals were sacrificed to remove the hearts, and the hearts were fixed with 10% neutral buffered formalin. These heart samples were cut into round slices, embedded in paraffin, the sliced sections were obtained, and after HE staining, the thickness of the anterior wall of the left wall, which was the site of myocardial infraction, was measured.

(i) Preparation of cell sheets

ADPMCs or bone-marrow-derived mesenchymal stem cells were cultured at 37°C for 72 hours in a medium containing IL-Iβ at a concentration of 5ng/mL in temperature-sensitive culture dishes. Cells were peeled off by incubating 20°C or lower for 30 minutes to obtain cell sheets. The obtained sheets were used in transplantation experiments described below.

(ii) Transplantation of sheets to myocardial infraction model rats

A myocardial infarction model rat was created by ligating the coronary arteries of nude rats. Two weeks later, the chest was opened again, and two cell sheets each, containing ADMPCs or bone marrow-derived mesenchymal stem cells cultured with or without IL-1β, were transplanted into the injured area. In the control group, sham surgery was performed with only thoracotomy.

(iii) Analysis of cardiac tissue to which sheets were transplanted

Rats were euthanized 4 weeks after transplantation and the hearts were removed. The removed hearts were fixed with 4% paraformaldehyde solution which was then replaced with 70% ethanol. The fixed hearts were cut into a few millimeters wide and hardened with paraffin to make blocks. The obtained paraffin blocks were sliced into 3 μm using a microtome, attached to slide glasses, and dried. The obtained thin sections were stained with hematoxylin eosin as follows.

(iv) Hematoxylin eosin staining

The thin sections were deparaffinized and washed with water. The sections were stained with hematoxylin solution for 10 minutes and washed with warm water for 3 minutes. After washing with water, the sections were stained with eosin for 5 minutes. The sections were dehydrated with alcohol. After being transparentized with xylene, the sections were sealed and observed under a microscope. The wall thickness of the anterior wall of the left ventricle, which is the infarcted area, was evaluated.

(2) Results of experiment

As shown in Fig. 6, in the group to which the sheets of IL-1β-treated ADMPCs were transplanted, or the group to which the sheets of IL-1β-treated bone marrow-derived mesenchymal stem cell were transplanted, wall thickness of the anterior wall of the left ventricle was improved, compared with the group to which the sheets of IL-1β-untreated ADMPCs were transplanted, or the group to which the sheets of IL-1β-untreated bone marrow-derived mesenchymal stem cell were transplanted.

These results indicate that IL-1β-treated ADMPCs or bone marrow-derived mesenchymal stem cells heal cardiac tissue damaged by severe myocardial infarction and significantly improve cardiac function.

Example 6

Example 6. Effect of treating adhesive cells derived from various mesenchymal tissues with various physiologically active polypeptides

(1) Method of experiment

As test cells, umbilical cord-derived mesenchymal stem cells (umbilical cord-derived MSCs), adipose tissue-derived stem cells (ADSCs), knee cartilage synovium-derived mesenchymal stem cells (synovium-derived MSCs), adipose tissue-derived multilineage progenitor cells (ADMPCs), placenta and its appendages-derived mesenchymal stem cells (placenta and its appendages-derived MSCs) and bone marrow-derived mesenchymal stem cells (bone marrow-derived MSCs) were used. Various cytokines, chemokines, growth factors and hormones were used as physiologically active polypeptides.

When ADMPCs were used as test cells, cytokines (IL-1α, IL-1β, IL3 to IL35, oncostatin M, LIF, CNTF, CT-1, TNFα, TNPβ, BAFF, FasL, RANKL, TRAIL, INF-α, IFN-β, IFN-γ), chemokines (CCL1 to CCL28, CXCL1 to CXCL10), growth factors (AvinA, ANGPLT5, BD-2, BD-3, BDNF, BMP-1 to BMP-7, DKK1, EGF, EG-VEGF, FGF-1 to FGF-21, G-CSF, HGF, IGF-1, IGF-2, PDGF-AA, PDGF-BB, R-spondin-1, R-spondin-2, R-spondin-3, SCF, galectin 1, galectin 2, galectin 3, GDF-11, GDNF, pleiotrophin, TGFα, TGFβ, TPO, TSLP, VEGF), and hormones (calcitonin, parathormone, glucagon, erythropoietin, leptin, ANP, BNP, CNP, oxytocin, vasopressin, TGH, TSH, CRH, ACTH, GRH, FSH, LH, SOM, GRH, GH, PRH, prolactin) were used as physiologically active polypeptides. When ADSCs, placenta and its appendages-derived MSCs, synovium-derived MSCs, bone marrow-derived MSCs and umbilical cord-derived MSCs were used as test cells, IL-1α, IL-1β, TNFα, TNPβ, IFN-β, IFN-γ, FGF15 were used as physiologically active polypeptides. Hereinafter, the physiologically active polypeptide is referred to as "drug".

The test cells were subjected to medium replacement with a drug-containing medium (final concentration of 100 ng/mL) and drug-free medium (control), and further subcultured for 3 days (72 hours). After 72 hours of medium change, 600 μL of RLT Buffer was added for recovery and RNA extraction.

As to RLT Buffer samples, total RNA was extracted using RNeasy Mini Kit/QIAGEN, and the total RNA was prepared in a concentration of 100 ng/μL. Then, labeled cRNA was synthesized from 150 ng of the total RNA per array. For the synthesized labeled cRNA, the concentration, yield and Cy3 uptake rate were calculated and the synthetic size (200 to 2000 nt were amplified) was measured. Six hundred ng of the labeled cRNA was fragmented at 60°C and hybridized at 65°C for 17 hours, and the array was washed and scanned.

A probe with the measured value reliable was extracted under the condition of either the control sample or the drug-added sample (one type), and the probe having an expression difference of 15 times or more was extracted as compared to the control sample.

(2) Results of experiment

Tables 2 to 7 show mRNAs whose expression was increased by 15 times or more after treatment with the drug as compared to those before treatment, and their multiplication factor.

[Table 2]

| UMBILICAL CORD-DERIVED MSCs | | |
|---|---|---|
| DRUG | INCREASED GENE | MULTIPLICATION FACTOR |
| TNFα | CSF2 | 51.92866 |
| IFNγ | SPARCL1 | 24.166359 |
| IL1α | CSF2 | 65.41428 |
| | CCL3 | 44.713257 |
| | MMP3 | 17.676903 |
| IL1β | CSF2 | 45.73727 |
| FGF15 | MMP7 | 20.78586 |

[Table 3]

| ADSCs | | |
|---|---|---|
| DRUG | INCREASED GENE | MULTIPLICATION FACTOR |
| TNF-α | CSF3 | 457.4336 |
| | CSF2 | 210.81802 |
| | MMP9 | 36.619884 |
| | LIF | 28.586313 |
| | FGF13 | 26.435118 |
| | BMP2 | 24.754152 |
| | MMP3 | 16.642172 |
| TNF-β | MMP9 | 50.442356 |
| | CSF3 | 23.138222 |
| | FGF5 | 15.041612 |
| IFNβ | CSF3 | 81.64009 |
| | CSF2 | 81.4202 |
| | BTC | 37.966434 |
| | FGF20 | 33.667175 |
| IFNγ | FGF20 | 17.39512 |
| | MMP25 | 15.073852 |
| IL-1α | CSF2 | 3650.884 |
| | CSF3 | 3004.3464 |
| | MMP3 | 153.17429 |
| | MMP12 | 48.928066 |
| | LIF | 44.622696 |
| | NTN1 | 19.101036 |
| | HBEGF | 17.43808 |
| | MMP1 | 16.788137 |
| | MMP8 | 15.74857 |

(continued)

| ADSCs | | |
|---|---|---|
| DRUG | INCREASED GENE | MULTIPLICATION FACTOR |
| IL- 1β | CSF3 | 3658.2717 |
| | CSF2 | 2945.6265 |
| | MMP3 | 163.10434 |
| | MMP12 | 77.35472 |
| | LIF | 44.191887 |
| | MMP8 | 20.121588 |
| | HBEGF | 17.731503 |
| | MMP1 | 17.499699 |
| | ADAMTS8 | 16.701633 |
| | FGF13 | 15.317384 |
| FGF15 | CSF3 | 1992.8231 |
| | CSF2 | 177.31819 |
| | MMP3 | 87.44734 |
| | MMP12 | 18.993986 |

[Table 4]

| SYNOVIUM-DERIVED MSCs | | |
|---|---|---|
| DRUG | INCREASED | MULTIPLICATION |
| TNFα | CSF3 | 481.9497 |
| | MMP3 | 184.5146 |
| | MMP1 | 118.73493 |
| | CSF2 | 93.68834 |
| | RSPO3 | 78.78901 |
| | RSPO3 | 54.598293 |
| | MMP12 | 37.193733 |
| | ANGPTL1 | 29.710234 |
| | LIF | 19.802446 |
| TNF-β | CSF3 | 133.98564 |
| | MMP3 | 79.96354 |
| | MMP1 | 57.18347 |
| | RSPO3 | 29.265568 |
| | RSPO3 | 22.514166 |
| IFNβ | ANGPTL1 | 30.57255 |
| | FGF20 | 17.808767 |
| IFN-γ | MMP25 | 18.338886 |

14

(continued)

| SYNOVIUM-DERIVED MSCs | | |
|---|---|---|
| DRUG | INCREASED | MULTIPLICATION |
| IL-1α | CSF3 | 10575.728 |
| | MMP3 | 1345.4216 |
| | MMP1 | 244.72272 |
| | MMP12 | 163.36778 |
| | CSF2 | 142.88773 |
| | MMP13 | 28.037321 |
| | MMP10 | 21.2812 |
| | RSPO3 | 18.53049 |
| IL1β | CSF3 | 8791.783 |
| | MMP3 | 935.04913 |
| | MMP12 | 181.83107 |
| | CSF2 | 129.32849 |
| | MMP1 | 107.597824 |
| | ADAMTS16 | 33.420284 |
| | GDF3 | 16.958546 |
| | MMP13 | 16.034931 |
| | IGF1 | 15.101902 |
| FGF15 | CSF3 | 1407.7273 |
| | MMP3 | 490.81107 |
| | MMP12 | 162.75064 |
| | MMP1 | 58.44772 |
| | IGF1 | 58.422787 |
| | BMP6 | 20.44636 |

[Table 5-1]

| ADMPCs | | |
|---|---|---|
| DRUG | INCREASED GENE | MULTIPLICATION FACTOR |
| BMP-3 | GCG | 17.480957 |
| | NRTN | 28.843102 |
| BMP-4 | REG4 | 75.89187 |
| BMP-6 | HDGFL1 | 65.01833 |
| CCL-3 | EGF | 15.73962 |
| CCL-5 | MMP26 | 29.093975 |
| | MMP13 | 17.447323 |
| CCL-8 | BMP7 | 17.2787 |
| CCL-9 | BMP10 | 28.345194 |

(continued)

| ADMPCs | | |
|---|---|---|
| DRUG | INCREASED GENE | MULTIPLICATION FACTOR |
| CCL-15 | BMP7 | 17.304893 |
| CCL-19 | FGF22 | 47.299706 |
| CCL-20 | EGF | 34.020695 |
| CCL-21 | BMP10 | 30.952303 |
| CCL-23 | ADIPOQ | 18.862783 |
| CCL-26 | FGF10 | 15.473124 |
| CCL-28 | NMU | 22.596947 |
| CNTF | ADAMTS20 | 44.9686 |
| | EGF | 20.999825 |
| CT-1 | FGF6 | 16.76011 |
| CXCL5 | ADAM22 | 15.880237 |
| CXCL10 | ADIPOQ | 23.21833 |
| | LTBP3 | 15.590775 |
| FGF15 | CSF3 | 78.26141 |
| | MMP3 | 21.577183 |
| GALECTIN 1 | IGF1 | 17.737694 |
| IFNβ | PTN | 34.09604 |
| | ANGPTL1 | 23.657429 |
| IFNγ | ADAMTS5 | 18.498682 |
| | GLDN | 93.92384 |
| | PGF | 33.75676 |
| | FGF21 | 16.46351 |
| IGF-2 | IGFL4 | 18.903763 |
| IL-1α | CSF3 | 1266.8649 |
| | CSF2 | 241.81323 |
| | MMP8 | 90.97638 |
| | MMP3 | 82.76877 |
| | MMP12 | 62.105858 |
| | FGF13 | 61.590416 |
| | MMP1 | 30.74703 |
| | MMP13 | 21.096134 |

[Table 5-2]

| DRUG | INCREASED GENE | MULTIPLICATION FACTOR |
|------|----------------|----------------------|
| IL-1β | CSF3 | 1414.8679 |
| | CSF2 | 215.49258 |
| | MMP8 | 82.693 |
| | MMP3 | 81.932846 |
| | MMP12 | 80.88991 |
| | FGF13 | 53.733124 |
| | MMP1 | 29.282549 |
| | MMP13 | 19.679766 |
| IL-7 | BMP3 | 15.619323 |
| IL-8 | EGF | 166.93967 |
| | ADAMTS20 | 27.746012 |
| | MMP7 | 15.016879 |
| IL-11 | OOSP2 | 27.152683 |
| IL-17 | CSF3 | 47.33713 |
| IL-24 | FGF2 0 | 16.994074 |
| IL-26 | MMP26 | 25.349789 |
| IL-31 | ADAM21 | 16.247 |
| IL-35 | NPPC | 27.392384 |
| | EPO | 21.361319 |
| TNFα | CSF3 | 760.7263 |
| | FGF13 | 519.2593 |
| | MMP8 | 322.53482 |
| | CSF2 | 84.63178 |
| | MMP3 | 42.06483 |
| | MMP1 | 41.600243 |
| | MMP13 | 33.378723 |
| | MMP10 | 33.15327 |
| | FGL2 | 30.280602 |
| | ADAM8 | 28.907818 |
| | MMP9 | 24.30117 |
| | RSPO3 | 22.690266 |
| TNFβ | FGF13 | 52.990948 |
| | CSF2 | 27.081493 |
| | CSF3 | 25.747252 |
| | MMP8 | 23.552876 |
| | MMP9 | 18.909662 |
| | MMP1 | 18.20611 |
| | MMP3 | 15.417225 |

(continued)

| DRUG | INCREASED GENE | MULTIPLICATION FACTOR |
|------|----------------|----------------------|
| TRAIL | OOSP2 | 30.486296 |

[Table 6]

| PLACENTA AND ITS APPENDAGES-DERIVED MSCs | | |
|------|----------------|----------------------|
| DRUG | INCREASED GENE | MULTIPLICATION FACTOR |
| TNFα | CSF2 | 359.60034 |
|  | MMP1 | 157.07306 |
|  | FGF13 | 138.73257 |
|  | CSF3 | 52.898098 |
|  | MMP9 | 52.12447 |
|  | RSPO3 | 36.97124 |
|  | MMP12 | 28.326378 |
|  | LIF | 20.185322 |
| TNPβ | CSF2 | 116.80491 |
|  | MMP1 | 35.85062 |
|  | RSPO3 | 33.528667 |
|  | FGF13 | 30.268114 |
| IFNβ | ANGPTL1 | 67.8647 |
| IL1α | CSF3 | 4009.4556 |
|  | CSF2 | 925.7604 |
|  | MMP1 | 86.76511 |
|  | MMP12 | 44.755383 |
|  | EGF | 32.053967 |
|  | MMP3 | 30.25197 |
|  | MMP20 | 19.222319 |
| IL1β | CSF2 | 2408.85 |
|  | CSF3 | 6519.517 |
|  | EGF | 24.028 |
|  | FGF13 | 18.65617 |
|  | MMP1 | 143.90538 |
|  | MMP12 | 87.97726 |
|  | MMP3 | 50.21765 |
| FGF15 | CSF3 | 653.6217 |
|  | CSF2 | 235.56853 |
|  | MMP1 | 16.586615 |

[Table 7]

| BONE MARROW-DERIVED MSCs | | |
|---|---|---|
| DRUG | INCREASED GENE | MULTIPLICATION FACTOR |
| TNFα | MMP1 | 62.074005 |
| | MMP3 | 39.413765 |
| | CSF2 | 34.723072 |
| | RSPO3 | 20.590866 |
| TNPβ | MMP1 | 16.974045 |
| IFNβ | BTC | 23.89296 |
| | DLL1 | 20.705479 |
| | PTN | 16.424856 |
| IFNγ | ANGPTL5 | 98.50543 |
| | RSP03 | 17.248734 |
| IL1α | MMP3 | 545.55664 |
| | CSF2 | 248.18904 |
| | MMP12 | 81.264694 |
| | MMP1 | 20.65767 |
| | CSF3 | 17.709955 |
| IL1β | CSF2 | 544.1642 |
| | MMP3 | 482.9409 |
| | MMP12 | 55.837635 |
| | NTN1 | 34.482384 |
| | MMP1 | 28.635344 |
| | CSF3 | 17.319433 |
| FGF15 | MMP3 | 46.422943 |
| | MMP1 | 21.592436 |

In any of the experiments, it was confirmed that expression of polypeptides, growth factors and/or enzymes involved in tissue healing was enhanced in adherent cells derived from mesenchymal tissue treated with a physiologically active polypeptide. In many combinations of the physiologically active polypeptide and adherent cells derived from mesenchymal tissue, CSF2 and/or CSF3 tended to be expressed, in particular highly expressed. From these results, in the present invention, it was found that a wide variety of physiologically active polypeptides and adherent cells derived from mesenchymal tissue can be used.

Example 7

Example 7. In vivo tissue healing effect of supernatant of ADMPCs treated with IL-1β - alleviation of liver tissue injury

(1) Method of experiment

Collection of adipose tissue from a human subject, isolation and culture of ADMPCs, and IL-Iβ treatment were performed in the same manner as in Example 2.
Culture supernatant of ADMPCs treated with IL-Iβ was concentrated by centrifugation for 30 minutes using Amicon Ultra-15 centrifugation filter unit, 3,000 NMWL. Fifty μL/animal of the concentrated supernatant was injected from via tail vein to NASH model mice (STAM (registered trade mark) mice), and liver tissue healing was investigated.

Animals were divided into the following two groups: a group to which the culture supernatant of ADMPCs treated with IL-Iβ was administered (n = 10), and a group to which a carrier was administered (n = 9). At the beginning of the study, streptozotocin was intradermally administered to animals in each group, and the animals were fed with a normal diet, fed with a high-fat diet from week 4 to week 9, and euthanized at week 9. Administration of the culture supernatant of ADMPCs treated with IL-1β and the carrier was performed once on week 6. The obtained liver tissue sections were subjected to Sirius red staining and hematoxylin-eosin (HE) staining.

(2) Results of experiment

(i) Sirius red staining of liver tissue sections

The results of Sirius red staining of liver tissue sections from mice obtained at week 9 of the study are shown Table 8. In the livers from mice to which the culture supernatant of ADMPCs treated with IL-Iβ was administered, it was confirmed that deposition of intrahepatic fibers stained with Sirius red was reduced, compared to the livers from mice to which the carrier was administered.

[Table 8]

| carrier group | culture supernatant of IL-Iβ treated ADMPC group |
|---|---|
| Sirius red staining fibrosis region (%) | |
| 0.88 | 0.68 |
| 1.02 | 0.53 |
| 0.79 | 0.74 |
| 0.98 | 0.33 |
| 1.24 | 0.61 |
| 1.03 | 0.58 |
| 0.67 | 0.70 |
| 0.81 | 0.72 |
| 0.92 | 0.26 |
| | 0.43 |
| average ± standard deviation | |
| 0.93±0.17 | 0.57±0.15 |
| Mann Whitney's U test p=0.000944 | |

(ii) Evaluation of liver tissue healing effect by NAFLED activity score (E. M. Brunt et al. Hepatology. 2011 March; 53(3): 810-820)

The degree of liver tissue injury in mice obtained at week 9 of the study was evaluated according to the NAFLED activity score. The evaluation method of NAFLED activity score is shown in Table 1 of Example 2.
The results are shown in Table 9. Because the NAFLD activity score was significantly lower in the livers from mice to which the culture supernatant of ADMPCs treated with IL-1β was administered, compared to the livers from mice to which the carrier was administered, it was confirmed that injury to liver tissue represented by vacuolation, inflammation and fatty change was greatly reduced.

[Table 9]

| carrier group | culture supernatant of IL-Iβ treated ADMPC group |
|---|---|
| NAFLD Activity Score | |
| 4 | 4 |
| 4 | 2 |

(continued)

| carrier group | culture supernatant of IL-Iβ treated ADMPC group |
|---|---|
| 5 | 4 |
| 4 | 2 |
| 5 | 3 |
| 5 | 5 |
| 4 | 2 |
| 4 | 2 |
| 4 | 5 |
|  | 4 |
| median | |
| 3 | 2 |
| Median test p=0.00712 | |

From these results, the culture supernatant of ADMPCs treated with IL-Iβ was found to be effective in healing injured tissue, and be useful for tissue protection, repair of tissue/cell injury, suppression of tissue inflammation and promotion of proliferation of cells constituting a tissue. It is considered that these tissue healings enables reconstruction of the tissue form and wound healing. Moreover, because these effects were observed in mice in which liver injury was induced by streptozotocin and high-fat diet, it can be said that IL-1β-treated ADMPCs are effective for tissue healing in chronic phase disease.

Example 8

Example 8. In vivo tissue healing effect of supernatant of ADMPCs treated with IL-1β - alleviation of lung tissue injury

(1) Method of experiment

Collection of adipose tissue from a human subject, isolation and culture of ADMPCs, and IL-1β treatment were performed in the same manner as in Example 2.
Fibrosis was induced in mice lunges using bleomycin (BLM). A BLM solution was administered endotracheally to mice. In particular, on the day when a BLM solution was administered, under anesthesia with 3 mL/kg of a mixed anesthetic (medetomidine hydrochloride 0.3 mg/kg, midazolam 4 mg/kg, and butorphanol tartrate 5mg/kg) subcutaneously administered , the neck of mice was incised, and trachea were exposed. Then, a nozzle of a liquid endotracheal administration device (IA-1C, Penn Century) was inserted through the mouth, and after confirming that it was in the trachea, a BLM solution (14 μg/25 μL/lung) was injected. Then, the incision was sutured, and 3 mL / kg of an α2 adrenergic receptor antagonist (atipamezole hydrochloride 2 mg/kg) was subcutaneously administered.
On the 21st day from BLM administration (the day of BLM administration was day 1 of administration), IL-1β treated ADMPCs and culture supernatant thereof were administered to the test groups in the tail vein while physiological saline was administered to the control group.
On the 35th day of BLM administration, the lungs were removed after being euthanized by exsanguination from the abdominal aorta under isoflurane anesthesia. The removed lung was expanded and fixed with 10% neutral buffered formalin at a water column pressure of 20 cm, and then fixed and stored with 10% neutral buffered formalin solution.
A formalin-fixed lung was cut out as shown in FIG. 9, a Masson's Trichrome (MT) stained specimen was prepared, and fibrosis was scored by classifying the degree of each lobe of the lung according to the evaluation criteria shown below. The evaluation criteria for pulmonary fibrosis are shown in Table 10.

[Table 10]

Evaluation criteria for pulmonary fibrosis (values are scores)
0: Normal
1: Mild fibrous thickening of the alveoli or bronchial walls is observed

(continued)

Evaluation criteria for pulmonary fibrosis (values are scores)

2: Moderate fibrous thickening of the alveoli or bronchial walls, however without overt structural changes in the lungs

3: Obvious structural changes in the lungs and formation of small fibrotic foci

4: Strong structural changes in the lungs and formation of large fibrotic foci

5: The whole lungs are replaced by fibrosis

The weighted averages were calculated from the specimens of the anterior lobe, middle lobe, posterior lobe, and accessory lobe of the left and right lungs, and used as pulmonary fibrosis scores. The pulmonary fibrosis scores shown in FIG. 10 were calculated using the following formula.

```
[Equation 1]

Weighted average of individual's pulmpnary fibrosis score =

[Left lung score + (Right lung anterior lobe score + Right lung middle lobe score + Right lung posterior lobe score +

Right lung accessory lobe score) / 4] / 2
```

(2) Results of experiment

As shown in FIG. 10, it was confirmed that ADMPCs treated with IL-1β and culture supernatant thereof are effective in healing lung tissue.

Industrial applicability

The present invention is useful in the field of pharmaceuticals for tissue healing and in the field of study of diseases which need tissue healing.

**Claims**

1.  A pharmaceutical composition for tissue healing, comprising adherent cells derived from mesenchymal tissue treated with a physiologically active polypeptide or lipopolysaccharide (LPS), or culture supernatant thereof, and a pharmaceutically acceptable carrier.

2.  The pharmaceutical composition according to claim 1, wherein the physiologically active polypeptide is one or more polypeptides selected from the group consisting of inflammatory cytokine, inflammatory cytokine-inducing polypeptide, growth factor, chemokine, hormone and interferon.

3.  The pharmaceutical composition according to claim 1 or 2, wherein the physiologically active polypeptide is one or more polypeptides selected from the group consisting of interferon-β (IFN-β), interferon gamma (IFNγ), interleukin-1 alpha (IL-1α), interleukin-1 beta (IL-1β), interleukin-17A (IL-17A), tumor necrosis factor alpha (TNFa), tumor necrosis factor beta (TNFβ), type I interferon (INF-I), transforming growth factor β (TGFβ), epidermal growth factor (EGF) and fibroblast growth factor (FGF).

4.  The pharmaceutical composition according to any one of claims 1 to 3, wherein the adherent cells derived from mesenchymal tissue are mesenchymal tissue-derived stem cells (MSCs), adipose tissue-derived multilineage progenitor cells (ADMPCs), placenta tissue-derived cells, umbilical cord tissue-derived cells, cells derived from tissue of placenta and its appendages, or bone marrow tissue- or synovium tissue-derived cells.

5.  The pharmaceutical composition according to any one of claims 1 to 4, wherein the tissue healing is tissue protection, repair of tissue/cell injury, promotion of proliferation of cells constituting a tissue, suppression of tissue inflammation or reconstruction of tissue form.

6. The pharmaceutical composition according to any one of claims 1 to 5, wherein the tissue healing is tissue healing in chronic phase disease.

7. A method for producing a pharmaceutical composition for tissue healing, comprising the steps of:

(a) treating adherent cells derived from mesenchymal tissue with a physiologically active polypeptide or LPS; and
(b) mixing the cells treated in step (a) or culture supernatant thereof with a pharmaceutically acceptable carrier.

8. The method according to claim 7, wherein the physiologically active polypeptide is one or more polypeptides selected from the group consisting of inflammatory cytokine, inflammatory cytokine-inducing polypeptide, growth factor, chemokine, hormone and interferon.

9. The method according to claim 7 or 8, wherein the physiologically active polypeptide is one or more polypeptides selected from the group consisting of interferon-$\beta$ (IFN-$\beta$), interferon gamma (IFN$\gamma$), interleukin-1 alpha (IL-1$\alpha$), interleukin-1 beta (IL-1$\beta$), interleukin-17A (IL-17A), tumor necrosis factor alpha (TNFa), tumor necrosis factor beta (TNF$\beta$), type I interferon (INF-I), transforming growth factor $\beta$ (TGF$\beta$), epidermal growth factor (EGF) and fibroblast growth factor (FGF).

10. The method according to any one of claims 7 to 9, wherein the adherent cells derived from mesenchymal tissue are mesenchymal tissue-derived stem cells (MSCs), adipose tissue-derived multilineage progenitor cells (ADMPCs), placenta tissue-derived cells, umbilical cord tissue-derived cells, cells derived from tissue of placenta and its appendages, or bone marrow tissue- or synovium tissue-derived cells.

11. The method according to any one of claims 7 to 10, wherein the tissue healing is tissue protection, repair of tissue/cell injury, promotion of proliferation of cells constituting a tissue, suppression of tissue inflammation, wound healing, or reconstruction of tissue form.

12. The method according to any one of claims 7 to 11, wherein the tissue healing is tissue healing in chronic phase disease.

[Fig. 1]

ADIPONECTIN

[Fig. 2]

**HGF**

[Fig. 3]

| CARRIER | hADMPC | IL-1β-TREATED hADMPC |

[Fig. 4]

| CARRIER | hADMPC | IL-1β-TREATED hADMPC |

[Fig. 5]

[Fig. 6]

ΔEF %

[Fig. 7]

[Fig. 8]

[Fig. 9]

A: trachea, B: bronchus, C: right lung, D: left lung,

1: anterior lobe, 2: middle lobe, 3: posterior lobe,

4: accessory lobe

[Fig. 10]

Pulmonary fibrosis grade

| | | | |
|---|---|---|---|
| 2.000 | | | |
| 1.900 | | | |
| 1.800 | | | |
| 1.700 | | | |
| 1.600 | | | |
| 1.500 | | | |

Control          IL-1β-treated          Culture supernatant of

ADMPC                      IL-1β-treated ADMPC

## INTERNATIONAL SEARCH REPORT

International application No.

PCT/JP2019/023873

### A. CLASSIFICATION OF SUBJECT MATTER
Int.Cl.    A61K35/28(2015.01)i,  A61K35/35(2015.01)i,  A61K35/50(2015.01)i,
           A61K35/51(2015.01)i,  A61L27/36(2006.01)i,  A61L27/38(2006.01)i,
           A61L27/40(2006.01)i,  A61P17/02(2006.01)i

According to International Patent Classification (IPC) or to both national classification and IPC

### B. FIELDS SEARCHED

Minimum documentation searched (classification system followed by classification symbols)
Int.Cl.    A61K35/28, A61K35/35, A61K35/50, A61K35/51, A61L27/36,
           A61L27/38, A61L27/40, A61P17/02

Documentation searched other than minimum documentation to the extent that such documents are included in the fields searched
Published examined utility model applications of Japan          1922–1996
Published unexamined utility model applications of Japan        1971–2019
Registered utility model specifications of Japan                1996–2019
Published registered utility model applications of Japan        1994–2019

Electronic data base consulted during the international search (name of data base and, where practicable, search terms used)
JSTPlus/JMEDPlus/JST7580 (JDreamIII), CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y<br>X | WANG, C. M. et al., "Co-treating mesenchymal stem cells with IL-1β and TNF-α increases VCAM-1 expression and improves post-ischemic myocardial function.", Mol Med Rep., 2014, vol. 10, pp. 792–798 (DOI: 10.3892/mmr.2014.2236) (abstract, pp. 794–796, fig. 5) | 1–12<br>1–12 |

☒  Further documents are listed in the continuation of Box C.          ☐  See patent family annex.

| | |
|---|---|
| *      Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| 30 July 2019(30.07.2019) | 13 August 2019 (13.08.2019) |

| Name and mailing address of the ISA/<br>    Japan Patent Office<br>    3-4-3, Kasumigaseki, Chiyoda-ku,<br>    Tokyo 100-8915, Japan | Authorized officer<br><br><br>Telephone No. |
|---|---|

Form PCT/ISA/210 (second sheet) (January 2015)

**INTERNATIONAL SEARCH REPORT**

International application No.

PCT/JP2019/023873

C (Continuation).　DOCUMENTS CONSIDERED TO BE RELEVANT

| Category* | Citation of document, with indication, where appropriate, of the relevant passages | Relevant to claim No. |
|---|---|---|
| X<br>Y | JP 2010-505849 A (UNIVERSITY OF VIRGINIA PATENT FOUNDATION) 25 February 2010, (claims 2, 16, example 1) & WO 2008/060374 A2 (claims 2, 16, example 1) & US 2010/0111897 A1 & EP 2076275 A1 & KR 10-2009-0086066 A | 1-12<br>1-12 |
| X<br>Y | WO 2016/125582 A1 (NATIONAL UNIVERSITY CORPORATION TOKYO MEDICAL AND DENTAL UNIVERSITY) 11 August 2016 (claim 2, examples 1-17, paragraphs [0036], [0002]-[0006]) (Family: none) | 1-12<br>1-12 |
| X<br>Y | WO 2012/020566 A1 (ASAHIKAWA MEDICAL UNIVERSITY) 16 February 2012 (claims 5, 8, 13, 6, paragraph [0070], examples 2, 3, paragraph [0081]) & US 2014/0030239 A1 (claims 5, 8, 13, 6, paragraph [0091], examples 2, 3, paragraph [0099]) & EP 2604686 A1 | 1-12<br>1-12 |
| Y | WO 2008/153179 A1 (MATSUYAMA, Akifumi) 18 December 2008 (paragraphs [0002], [0004], claims, examples 3, 10) & US 2010/0151574 A1(paragraphs [0002], [0004], claims, examples 3, 10) & EP 2166084 A1 | 1-12 |
| Y | 松山晃文, 幹細胞の基礎研究 脂肪幹細胞の新展開 医学のあゆみ, 2009, vol. 229, no. 9, pp. 694-697 (ISSN:0039-2359) (abstract, p. 696, right column, paragraph [0002] to p. 697, left column), non-official translation (MATSUYAMA, Akifumi, "Basic research on stem cells, A novel adipose tissue-derived stem cell", Igaku no ayumi) | 1-12 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008153179 A

**Non-patent literature cited in the description**

- **ZUK PA ; ZHU M ; ASHJIAN P et al.** Human adipose tissue is a source of multipotent stem cells. *Mol Biol Cell,* 2002, vol. 13, 4279-4295

- *Japanese Journal of Transfusion and Cell Therapy,* 2013, vol. 59 (3), 450-456